Europäisches Patentamt

European Patent Office

Office européen des brevets

(11) Publication number: **0 260 856**

**A2**

(12) # EUROPEAN PATENT APPLICATION

(21) Application number: **87307882.8**

(22) Date of filing: **07.09.87**

(51) Int. Cl.⁴: **A61B 1/06** , G02B 6/00

(30) Priority: **19.09.86 US 909264**

(43) Date of publication of application:
**23.03.88 Bulletin 88/12**

(84) Designated Contracting States:
**DE FR GB**

(71) Applicant: **BAXTER TRAVENOL
LABORATORIES, INC.
One Baxter Parkway
Deerfield, IL 60015(US)**

(72) Inventor: **Forkner, John F.
31661 Florence Ave
SO. Laguna CA. 92677(US)**

(74) Representative: **Day, Jeremy John et al
REDDIE & GROSE 16 Theobalds Road
London, WC1X 8PL(GB)**

(54) **Distortion corrected endoscope.**

(57) An endoscope comprising a body including an elongated tubular distal section adapted for insertion into a patient and image-transmitting optics for transmitting an image through the distal section so that the image can be viewed at a viewing location. The image-transmitting optics include an elongated image-transmitting rod and a wedge surface lying at an acute angle with respect to the optical axis to provide an offset field of view. The acute angle of the wedge surface of the image-transmitting rod introduces asymmetric distortion to the image, and such distortion is reduced using one or more prisms.

FIG. 1

EP 0 260 856 A2

# DISTORTION CORRECTED ENDOSCOPE

An endoscope is used to enable a physician to view internal regions of a human or animal body. An endoscope may typically comprise a body, including an elongated tubular distal section adapted for insertion into a patient and a viewing port. Image transmitting means is provided within the body to transmit an image through the distal section so that the image can be viewed at the viewing port. The field of view can be illuminated in various ways, such as by optical illumination fibers extending through the distal section of the endoscope.

For some applications, such as arthroscopy, it is desirable to provide the endoscope with an offset field of view. By rotating the endoscope, the offset field of view is also rotated, and a much larger field of coverage is obtained. In some conventional endoscopes, such as arthroscopes and cystoscopes, the offset field of view is provided by a reflecting prism having a wedge surface inclined to the optical axis at the distal end of the scope. The offset field of view also reduces objectionable reflections from shiny surfaces. The image from the prism is then transmitted proximally using a lens system or an image-transmitting fiberoptic bundle.

Because of the need for multiple, accurately cut and polished surfaces and the need for reflective coatings, reflecting prisms are very difficult or impossible to make below certain diameters, and for this reason, are not well suited to endoscopes which must have a distal section of small diameter. In addition, reflecting prisms are very expensive to make in these small sizes.

## SUMMARY OF THE INVENTION

This invention provides a very simple and economical way for creating an offset field of view in an endoscope. In addition, the distortion of the image introduced by the means for providing the offset field of view is significantly reduced by this invention. Although these features are specifically applicable to endoscopes, in a more general sense, they are broadly applicable to micro-telescopes which can be used to visually examine inaccessible regions in manufactured parts and elsewhere.

According to one feature of the invention, the reflecting prism of the prior art is replaced with a refracting prism. This refracting prism is preferably, although not necessarily, constructed of gradient index material. Gradient index material can be relatively inexpensively provided in long, image-transmitting rods. Accordingly, an image-transmitting rod of gradient index material can be relatively easily cut at an acute angle with respect to its longitudinal axis to provide a wedge surface at the distal end which provides an offset field of view. This is very easy to make and inexpensive when compared with the reflecting prism of the prior art.

When provided in this form, the gradient index material forms a refracting prism. A refracting prism obtains an offset field of view by refracting the centerline of the offset field of view, whereas a reflecting prism does not.

To minimize cost and facilitate manufacture, the refracting prism is preferably constructed of gradient index material. However, the refracting prism can be constructed of glass if it is desired to increase the entrance pupil to collect more light. In either event, the refracting prism is less expensive and easier to make than the reflecting prism because a refracting prism has no reflective coatings, and it has fewer cut and polished surfaces.

The image from the refracting prism can be transmitted proximally using a lens system or an image-transmitting rod. As used herein, the image-transmitting rod includes any rigid or flexible rod, strand or cable-like member that transmits an image, such as image-transmitting rods of gradient index material or one or more image-transmitting optical fibers. The image-transmitting rod may comprise one or more rod sections. In a preferred construction, the image is transmitted proximally by a relay rod of gradient index material.

The image-transmitting means transmits the image proximally to a viewing location which may be a viewing port at which the image can be directly viewed by the observer or a location from which the image is further transmitted, such as by optics and/or electronics, for remote viewing. For example, a T.V. camera or other camera may be at the viewing location. The image-transmitting means includes, not only the image-transmitting rod, but an objective lens to form an image at the distal end of the image-conducting rod. In a preferred construction, the objective is formed by gradient index material having a pitch length which is shorter than the pitch length of the relay rod.

The image-transmitting means preferably includes means to enlarge the image near the proximal end of the image-transmitting means, and this function can be provided by a conventional eyepiece lens. Alternatively or in addition thereto, this image-enlarging function can be performed by the relay rod by providing it with a length approximately equal to N/4 pitches where N is an odd integer. The reason for this is that the rays in the rod become approximately parallel as they exit from a rod of this length.

Although illumination can be provided in different ways, preferably optical illumination fibers extend along at least a region of the image-transmitting means in the distal section of the endoscope body for conducting light to a region adjacent the distal end of the image-transmitting rod. The fibers have distal ends which lie at an acute angle with respect to the optical axis to direct the illumination in the same general direction as the field of view. Preferably, the acute angle at which the fibers are cut is substantially equal to the acute angle of the wedge surface of the refracting prism.

Use of the wedge surface of a refracting prism to create the offset field of view introduces distortion to the image and poor resolution, and this invention reduces such distortion and improves resolution. The distortion is assymetric and includes compression of one side of the image. To correct for this, the distortion-reducing means includes means for differentially magnifying the image. If it is desired to have the distal section be disposable, the distortion-reducing means is preferably located in an eyepiece housing which is reusable.

The distortion-reducing means can advantageously include one or more prisms for differentially magnifying the image in one plane. In a preferred construction, first and second prisms having different refracting indices and an interface for differentially magnifying the image in one plane are used. The use of first and second prisms tends to elongate the field of view in one direction, and to correct for this, the image-transmitting means preferably includes means for shortening the field of view in such direction. The shortening means may include, for example, an appropriate telescope.

One advantage of using first and second prisms is that the ratio of the refractive indices can be small; for example, down to 1.04, and this reduces the reflection loss at the interface. In addition, the prisms can be color-corrected if the Abbe number of the two prisms is substantially the same. Thus, the first and second prisms should have refractive indices which are slightly different, and Abbe numbers which are substantially identical.

The invention, together with additional features and advantages thereof, may best be understood by reference to the following description taken in connection with the accompanying illustrative drawing.

## BRIEF DESCRIPTION OF THE DRAWINGS

Fig. 1 is a schematic view illustrating one form of endoscope constructed in accordance with the teachings of this invention.

Fig. 1a is an enlarged, fragmentary sectional view of the distal end portion of the endoscope.

Fig. 2 is a schematic illustration showing the transmission of a chief ray through the objective lens and the relay rod.

Fig. 3 is a fragmentary schematic view illustrating an alternative construction for the distal tip of the endoscope.

Fig. 4 is a schematic view similar to Fig. 2 illustrating the use of gradient index material to carry out an eyepiece function.

Fig. 5 is a schematic view of the distal end portion of the image-transmitting means illustrating how the wedge surface introduces distortion into the image.

Fig. 6 is a schematic illustration of one fixed circular field of view showing how the distortion caused by the wedge surface distorts elements or objects near the bottom of the field.

Figs. 7, 8 and 9 are schematic views illustrating different forms of distortion-correcting systems, together with associated optics.

Fig. 10 is a longitudinal, sectional view illustrating one form that the endoscope of this invention may take.

Fig. 11 is an enlarged, fragmentary view showing one way to releasably attach the distal section to the eyepiece housing.

Fig. 12 is a sectional view taken generally along line 11-11 of Fig. 10.

## DESCRIPTION OF THE PREFERRED EMBODIMENT

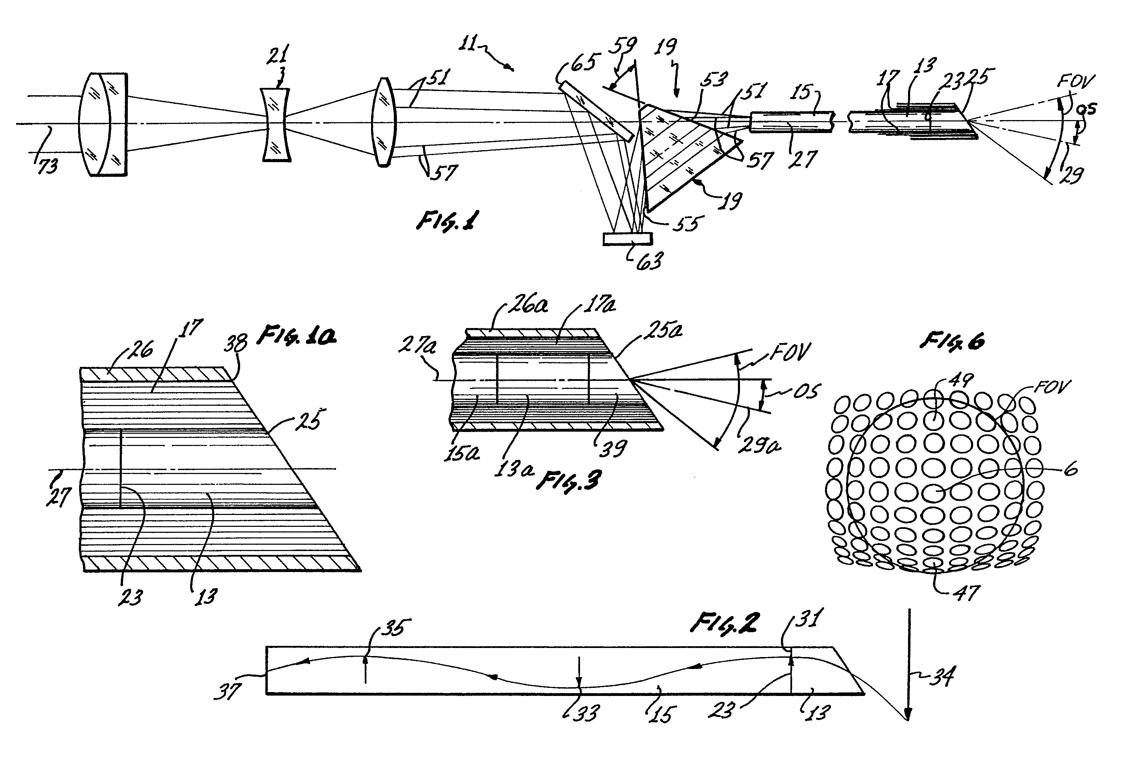

Fig. 1 shows an endoscope 11 which generally comprises an objective lens 13, an image-transmitting relay rod 15, illumination fibers 17, a distortion-correcting prism 19 and a conventional inverting eyepiece lens system 21. A tube 26 (Fig. 1a) houses the objective lens 13 and portions of the relay rod 15 and fibers 17. Although the system shown in Fig. 1 is referred to as an endoscope, it will be recognized that the same components can be used as a micro-telescope for purposes outside of the medical field.

In this embodiment, the objective lens 13 and the relay rod 15 are both constructed of gradient index material and may be, for example, about 1/2 millimeter in diameter. The objective lens 13 and the relay rod 15 may be cemented together along an interface 23.

The objective lens 13 has a distal end or wedge surface 25 which forms an acute angle with an optical axis 27, which coincides with the longitudinal axis of the objective lens 13 and the relay rod 15. Because the wedge surface 25 is formed at an acute angle with respect to the optical axis 27, the objective lens 13 also serves as a refracting prism which provides an offset field of view (FOV) as shown in Fig. 1. The field of view has a centerline 29 which is refracted by the wedge surface 25 of the objective lens 13 and an offset angle (OS) formed by the optical axis 27 and the centerline 29. Accordingly, by rotating the endoscope 11 about the optical axis 27, a larger viewing field is exposed to the observer.

As shown in Fig. 2, light is transmitted through the objective lens 13 and the relay rod 15 in sinusoidal fashion as represented by the chief ray shown in Fig. 2. A full 360 degrees of the sine wave represents one pitch, and images are formed at image planes 31, 33 and 35 as represented by the arrows in Fig. 2. The direction of the arrows indicate whether the image is erect or inverted with the downwardly-directed arrows representing an erect image of an object 34 (Figs. 2). The relay rod 15 has a distal end at the interface 23, and the image plane 31 is formed at the interface. The beam leaving the proximal face 37 of the relay rod is collimated, and to achieve this result, the relay rod has N/4 pitches where N is an odd integer. In Fig. 2, N is 5, but this is merely illustrative, and the image is inverted. The conventional inverting eyepiece system 21 provides an erect image for the observer following distortion correction.

The objective lens 13 covers a relatively wide angle and forms an image at the image plane 31 at the interface 23. For this purpose, it is relatively short and, in this embodiment, is slightly greater than 1/4 pitch. An objective lens of 1/4 pitch will focus parallel rays from infinity, and an objective lens of greater than 1/4 pitch will focus rays from a nearer location at the interface 23. The relay rod 15 is typically much longer than the objective lens 13 so it can relay the image at the interface 23 to or toward the viewer or other means, such as a camera, which will transmit the image to the viewer.

As shown in Fig. 1a, the illumination fibers 17 have distal ends 38 which are cut at, and lie at, an acute angle with respect to the optical axis 27, and the distal ends 38 lie in substantially the same plane as the wedge surface 25. This directs illumination toward the offset field of view (FOV). To support and house the fibers 17, the distal end of the tube 26 is cut at the same angle and is coplanar with the wedge surface 25.

A modification of the distal region of the endoscope 11 is shown in Fig. 3. In Fig. 3, the objective lens 13a and the relay rod 15a are identical to the corresponding components in Fig. 1, except that the objective lens 13a does not also serve as a refracting prism. Rather, a separate refracting prism 39 of glass is formed with a wedge surface 25a corresponding to the wedge surface 25 of Fig. 1 which refracts the centerline 29a of the field of view (FOV) away from the optical axis 27a. The prism 39 may be attached to the distal end of the objective lens 13a in coaxial alignment therewith. The use of the prism 39 is desirable when a larger entrance pupil is needed to collect more light. In all other respects, the embodiment of Fig. 3 may be identical to the embodiment of Fig. 1, and in this regard, illumination fibers 17a extend within the tube 26a to provide illumination for the field of view as discussed above.

Fig. 4 shows another variation in which the relay rod is constructed to perform an image-enlarging function, such that the eyepiece system 21 can be eliminated if desired. The modification of Fig. 4 can be used with or without distortion correction. Except as noted herein, the embodiment of Fig. 4 may be identical to the embodiment of Fig. 1, and corresponding portions are designated by corresponding reference numerals followed by the letter "b."

Fig. 4 shows how rays 41 and 43 come together at the image planes 31, 33, 35 and 36, and between image planes, the rays first diverge and then converge toward the next image plane. The relay rod 15b terminates at the proximal end 37b, which is normal to the optical axis 27b. Because the proximal end 37b is at N/4 pitches, where N is an odd integer, from the interface 23b, the rays 41 and 43 leave the proximal end 37b generally parallel. Because the light leaving the proximal end 37b is collimated, the end 37b can be brought close to the eye to achieve a magnification effect. More specifically, the proximal end 37 is at n + .75 pitches, where n is an integer, from the interface 23 so that the image is erect, and inverting optics, such as an inverting eyepiece, is not needed.

The image transmitted by the relay rod 15 (Fig. 1) is distorted, and although the distortion-correcting prism 19 is optional, its use is highly desirable, particularly at larger acute or wedge angles W (Fig. 5) of the wedge surface 25. Essentially all of the distortion of the image is generated at the wedge surface 25. This distortion results from the non-linear characteristic of Snell's Law, which is the basic law that applies at all refracting surfaces. The non-linear refracted ray angles at the wedge surface 25 result in a variation of angular magnification across the field of view, corresponding to varying angles of incidence at the wedge surface. An equation for the variation of magnification with incidence angle can be obtained by differentiating Snell's Law of Refraction to yield:

$$M = dI'/dI = (N\cos I)/(N'\cos I')$$

where M is magnification, N is the refractive index before refraction, e.g. in saline solution, N' is the refractive index after refraction, i.e. of the objective lens 13, I is the incidence angle of any ray 45 which forms an angle U with the optical axis 27 and I' is the refraction angle as shown in Fig. 5.

From the above equation, it is apparent that equal size objects at different locations across the field tend to become compressed where the incidence angles at the wedge surface 25 are large and that very little distortion occurs on the opposite side of the field where the incidence angles are small. This compression of one side of the image increases with wedge angle W, and the distortion is probably acceptable for small wedge angles, such as 17 degrees. However, the offset field of view for a 1.7-degree wedge angle W is only 5.5 degrees, which is rather small, and only increases the conical field covered by 11 degrees as the result of rotation of the endoscope 11. For those cases in which greater coverage is desired, distortion correction becomes more desirable. Thus, for wedge angles W of the order of 35 degrees, distortion correction is highly desirable.

Fig. 6 illustrates the general nature of the distortion for one fixed field of view of the endoscope 11 for a wedge angle W of 36.6 degrees, an equivalent offset angle in water of 29.6 degrees and a field of view angles of 44.1 by 64.8 degrees. As represented in Fig. 6, a segment or element 47 near the bottom of the field of view is compressed vertically along the "Y" axis, whereas a segment or element 49 near the top of the field of view is essentially undistorted. The distortion ratio, which is the vertical dimension of the element 47 divided by the vertical dimension of the element 49 is .26. The elements 47 and 49 are of equal size, if undistorted.

The prism 19 compensates for the distortion produced at the wedge surface 25 by using the same type of refractive distortion that occurs at the wedge surface. However, in the correcting mode, the distortion is used in a reverse order. Because of the relatively large refraction angles involved in the distortion-correcting prism 19, the prism 19 receives collimated light in order to avoid excessive astigmatism and other aberrations. In this embodiment, the collimated light is provided by the relay rod 15, although other means for providing collimated light to the prism 19 can be employed, if desired.

Generally, the correcting prism 19 serves as an anamorphic telescope and elongates the compressed portions of the image and leaves the relatively undistorted parts of the image unchanged. The prism 19 is oriented so that rays 51 (Fig. 1) coming from the undistorted side of the field, e.g. from the element 49, strike a planar prism surface 53 at the incidence angle for a minimum angle of deviation condition, which is often encountered in prism spectrometers. At the minimum deviation condition, the incidence angle at the prism surface 53 equals the exiting refracted angle at a planar prism surface 55. Accordingly, the prism 19 reproduces the angular dimensions of the object at unit magnification. Application of the magnification equation given above at the surfaces 53 and 55 would yield magnifications at the surfaces that are reciprocals of each other, and when multiplied together, give a total prism magnification of unity.

At the opposite side of the field, e.g. from the segment 47, rays 57 strike the prism surface 53 at an incidence angle which does not equal the exit refracted angle, and a net magnification by the prism 19 results. In other words, the element 47 (Fig. 6) is stretched along the "Y" axis, and the element 49 is not magnified. Of course, the magnification effect obtained by the prism is in only one plane. In designing the prism 19, the angle 59 defined by the surfaces 53 and 55 and its orientation to the optical axis 27 are adjusted so that the distortion across the field is minimized.

The relay rod 15 and the objective lens 13 serve as a telescope having a power equal to the ratio of the length of one pitch length of the relay rod to one pitch length of the objective lens. For a five-power telescope with no distortion, a five-degree object angle would be reduced to one degree after being relayed by the relay rod. However, with the distortion provided by the wedge angle W, if the elements or objects 47 and 49 and a central element 61 (Fig. 6) are five-degree objects, they may appear after relaying as, for example, .4, 1.093 and .948 degree images, respectively.

5

The distortion correction provided by the prism 19 is not perfect; however, it does represent a very substantial improvement and reduction in the distortion. If the elements 47, 49 and 61 (Fig. 6) were 5 degree objects, a perfect distortion correction system for the above example would yield 1 degree object angles after correction. For example, after correction, the elements 47, 49 and 61 (Fig. 6) may have object angles of 1.27 degrees, .904 degrees and 1.24 degrees, respectively. The distortion correction slightly increases the object angle for the central element 61. However, overall the image provided by the distortion-correcting prism 19 is a vast improvement over the distorted image.

The image-transmitting means also includes flat mirrors 63 and 65, which reflect the rays 51 and 57 back along the optical axis 27. Accordingly, the beam is coaxial with the optical axis 27. The eyepiece system 21 inverts and enlarges the image in a conventional fashion so that it can be viewed erect at a viewing location 73.

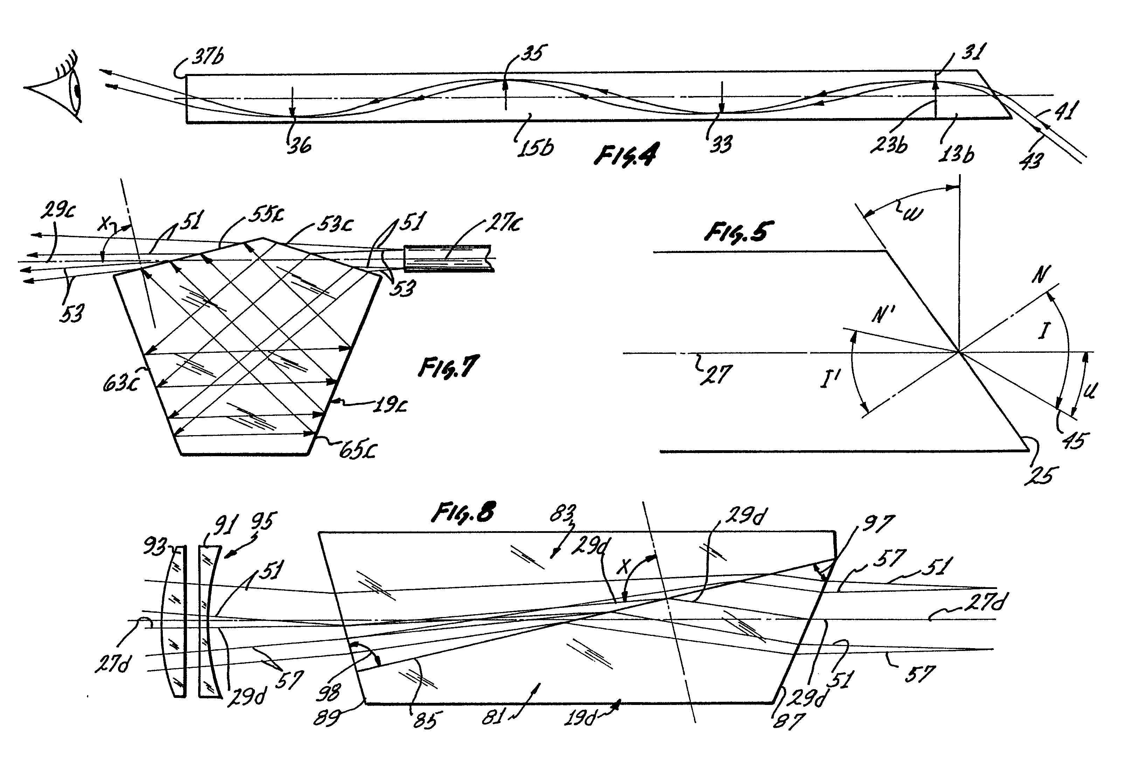

Fig. 7 shows a prism 19c which can be used in the endoscope 11 of Fig. 1 in place of the prism 19 and the mirrors 63 and 65. The prism 19c serves the combined functions of the prism 19 and the mirrors 63 and 65. Portions of the prism 19c corresponding to portions of the prism 19 and the mirrors 63 and 65 are designated by corresponding reference numerals followed by the letter "c." In the embodiment of Fig. 7, the mirrors 63c and 65c are flat surfaces of the prism 19c. The prism surfaces 53c and 55c perform the same function discussed above for the prism surfaces 53 and 55.

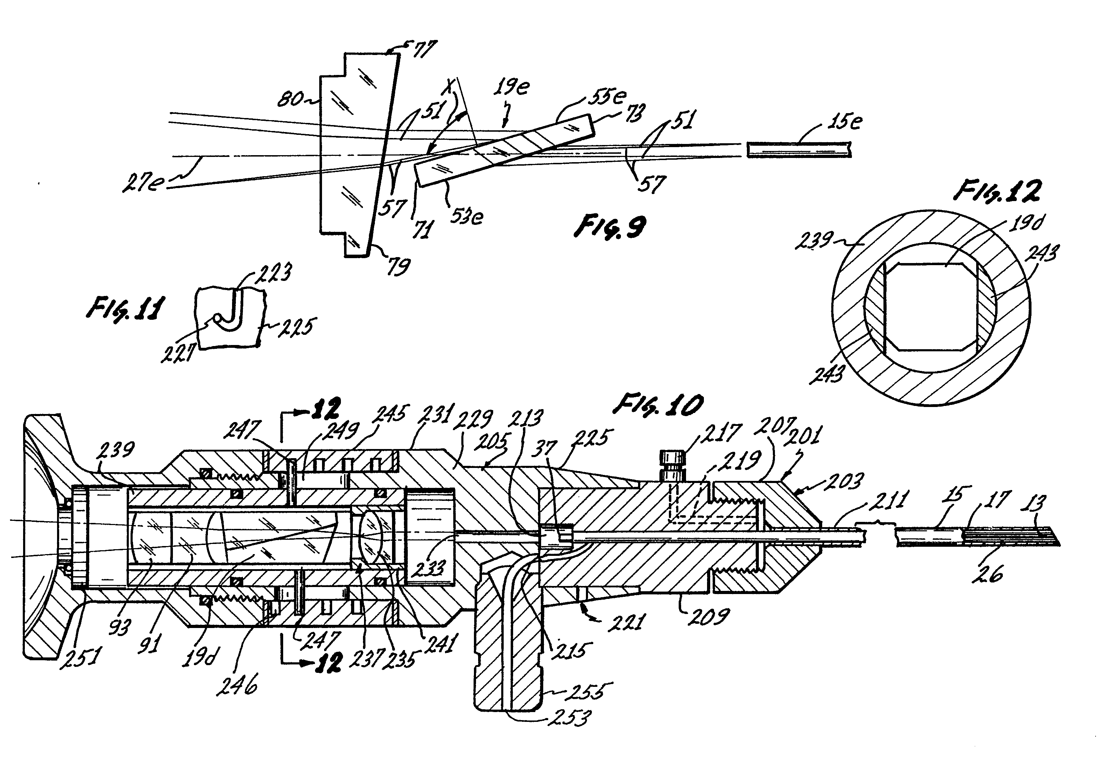

Fig. 8 shows the currently preferred form of distortion-correcting prism 19d which can be embodied in an endoscope as shown by way of example in Figs. 10-12. The prism 19d includes prisms 81 and 83 cemented together along a planar interface 85. The prism 19d has a planar entrance surface 87 and a planar exit surface 89.

The prisms 81 and 83 are constructed of different kinds of glass, with each of the prisms having a different refractive index. Preferably, the ratio of the refractive index of the prism 81 to the refractive index of the prism 83 is small and may be, for example, about 1.04. By keeping this ratio slightly greater than "1", the reflection loss at the interface 85 is reduced.

One problem is that the magnification obtained from an optical element, such as the prism 19d, changes with wavelength. Accordingly, color correction is desirable. It has been found that color correction can be obtained by selecting the glass for the prisms 81 and 83, such that the Abbe numbers are substantially the same. For example, suitable materials for the prisms 81 and 83 are identified in the table below:

| Glass Nos. | Refractive Index (N) | Abbe No. (V) |
|---|---|---|
| 607567 | 1.607 | 56.7 |
| 501564 | 1.501 | 56.4 |
| 564608 | 1.564 | 60.8 |
| 511604 | 1.511 | 60.4 |
| 510667 | 1.510 | 66.7 |
| Fused Silica | 1.458 | 67.9 |

In the above table, the Glass Nos. 607567, 564608 and 510667 identify glass suitable for the prism 81, and the other Glass numbers identify glass suitable for the prism 83. As shown by the above table, the Abbe numbers need not be identical, but the closer their numerical values, the greater is the color correction. Generally, suitable color correction can be obtained if the Abbe numbers are within two percent of each other, but preferably, the Abbe numbers are within .2 percent of each other. As used herein, Abbe numbers within .2 percent of each other are considered to be substantially the same.

In the prism 19d, the distortion correction is provided by the interface 85. The angles of the surfaces 87 and 89 are selected such that the beam will leave the exit surface 89 coaxial with the optical axis 27. Generally, the angle X (Fig. 8) between a reference line normal to the interface 85 and the centerline 29d of the field of view should be such that the desired nonlinear correction is obtained, and for this purpose, X should be within 70 and 88 degrees and is preferably between 80 and 85 degrees. Below 70 degrees, there is not much nonlinear effect, and above 88 degrees, mechanical difficulties arise. The angle X is also shown in Fig. 7 between a reference normal to the prism surface 55c and the centerline of the field of view, and the angle X also exists in Fig. 1.

As shown in Fig. 8, the rays 51 coming from the undistorted side of the field exit the exit surface 89 of the prism 19d as shown in Fig. 8, whereas the rays 57 are magnified as shown. In addition, the prism 19d stretches out the entire field of view, and in the configuration shown in Fig. 8, the field of view is stretched from 3.2 degrees at the entrance surface 87 to 8.7 degrees beyond the exit surface 89. A cylindrical planoconcave lens 91 and a cylindrical planoconvex lens 93 combine to form an anamorphic telescope 95 which provides the magnification necessary to reduce the angle back to, in this example, 3.2 degrees.

In the form shown in Fig. 8, the rays 51 from the undistorted side of the field come from the upper side of the field of view. Of course, the prism 19d must be arranged to provide the distortion correction from the applicable side of the field. Although various constructions are possible, by way of example, the interface 85 forms an angle 97 with the entrance surface 87 and an angle 98 with the exit surface 89.

Fig. 9 illustrates another system for correcting the distortion, and it can be used in the endoscope of Fig. 1 in lieu of the prism 19. The prism 19e has the advantages of being relatively inexpensive and easy to make, and it also reduces color aberration somewhat. Portions of the distortion-correcting apparatus shown in Fig. 9 corresponding to portions of the distortion-correcting apparatus of Fig. 1 are designated by corresponding reference numerals followed by the letter "e."

In Fig. 9, the prism 19e is in the form of a wedge in which the surfaces 53 and 55 diverge slightly as they extend downwardly to the left as viewed in Fig. 9 such that the prism 19e has a thicker end surface 71 and a thinner end surface 73, with the end surfaces 71 and 73 being parallel. For example, the surfaces 53e and 55e may diverge or form an acute angle of about 1 degree plus or minus .25 degree. The prism 19e is arranged at an acute angle relative to the optical axis 27e, and such acute angle may be, for example, about 15 degrees plus or minus 1 degree. These angular relationships are selected to provide the angle X within the range described above. However, in order to obtain some correction, the angle formed by the surfaces 53e and 55e must be greater than 0. Of course, the prism 19e and the distortion-correcting surfaces of the prisms of the other embodiments are inclined in the appropriate direction to correct the distortion rather than to magnify the distortion. The prism 19e, like the prism of the above-described embodiments, is sufficiently wide to fully intercept the beam which it receives from the relay rod 15e.

The prism 19e functions much like the prisms 19 and 19c, except that no reflecting surfaces are necessary. Thus, the prism 19e receives collimated light from the relay rod 15e and achieves differential magnification of the image in that the rays 57 from the lower or distorted side of the field are magnified, whereas the rays 51 from the undistorted side of the field are subjected essentially to unity magnification.

The prism 19e tilts the centerline 29e field of view slightly on the exit side of the prism. This can be corrected with a prism 77 having a planar entrance surface 79 which is inclined relative to the optical axis 27e, with the angle of inclination being sufficient to provide the correction desired. For example, the planar entrance surface 79 may form about a 10-degree angle with a plane normal to the optical axis 27e. The prism 77 has an exit surface 80 which may be perpendicular to the optical axis 27e. The beam from the prism 77 may be received by an eyepiece of suitable design.

The optical components described above can be embodied in an endoscope 201 (Fig. 10) which generally comprises a tubular, disposable distal section 203 and a reusable eyepiece housing 205. The distal section 203 includes the tube 26, a nut 207, a connector 209 and an irrigation cannula 211. The tube 26 houses the objective lens 13 and portions of the relay rod 15 and the illumination fibers 17 as described above in connection with Fig. 1a. The relay rod 15, which in this embodiment, is 4.25 pitches in length extends completely through the tube 26 and the nut 207 and terminates in the proximal surface 37 in a recess 213 in the connector 209 to provide a collimated exit beam. Similarly, the illumination fibers 17 extend completely through the tube 26 and the nut 207 and terminate at a proximal end 215 at the proximal end of the connector 209.

The irrigation cannula 211 surrounds the tube 26 so that irrigation fluid can be supplied to the distal end of the endoscope 201 from a fitting 217 through a passage 219 in the connector 209 to an annular space between the irrigation cannula 211 and the tube 26.

The nut 207 is threaded onto the connector 209, and the connector 209 is suitably releasably mounted on the eyepiece housing in any suitable manner, such as by a fastener 221 which releasably retains the connector 209 on the eyepiece housing in a predetermined angular orientation. For example, the fastener 221 may comprise detents or a bayonet-type fastener which includes a J-slot 223 in a tubular section 225 of the eyepiece housing 205 and a pin 227 carried by the connector 209 and receivable in the slot as shown in Fig. 11.

The eyepiece housing 205 includes a transverse wall 229 dividing the tubular section 225 from a tubular section 231. A relay rod 233 of gradient index material and of 1/4 pitch length is sealed in the transverse wall 229 to relay collimated light from the proximal face 37 of the relay rod 15 proximally to a conventional spherical achromatic lens 235. The achromatic lens 235, the prism 19d and the lenses 91 and 93 are housed within a holder 237 fixedly held within a sleeve 239 which is mounted for slidable movement in the tubular section 231. As shown in Figs. 10 and 12, the holder 237 includes a tubular section 241 for retaining the achromatic lens 235 and opposed leg sections 243 coupled to the tubular section 241 for retaining the prism 19d and the lenses 91 and 93. Of course, the holder 237 may retain other optical elements, and any of the other distortion-correcting prisms can be embodied in the endoscope 201, if desired.

The eyepiece housing 205 also includes a focusing ring 245 having an internal helical groove 246 for slidably receiving radial pins 247 which extend through axial slots 249 in the tubular section 231 and are mounted on the sleeve 239. Accordingly, rotation of the focusing ring translates the sleeve 239 and the pins 247 and moves the achromatic lens 235 toward and away from the relay rod 233 to obtain focusing. The sleeve 239 is held against rotation relative to the eyepiece housing 205 by the pins 247 and the associated slots 249.

The eyepiece housing 205 terminates proximally in a window 251 at a viewing location. Of course, a camera or other means may be provided at the viewing location to record and/or transmit the image. Alternatively or in addition thereto, the image may be transmitted optically to other locations.

Illumination can be provided to the illumination fibers 17 by optical fibers 253 which extend through an optical fiber connector member 255 and terminate in confronting relationship with the proximal ends 215 of the illumination fibers 17. With this construction, the distal section 203 can be removed from the eyepiece housing following usage of the endoscope 201 and replaced with a new distal section. This preserves the eyepiece housing and the more expensive components contained therein for subsequent use. The transverse wall 229 and the relay rod 233 sealed therein provide no opening for direct communication between the interior of the tubular section 231 and the distal section 203. Optically, the endoscope 201 functions as described above.

Although exemplary embodiments of the invention have been shown and described, many changes, modifications and substitutions may be made by one having ordinary skill in the art without necessarily departing from the spirit and scope of this invention.

**Claims**

1. An apparatus comprising:
a body including an elongated tubular distal section adapted for insertion into an patient and a proximal viewing location;
image-transmitting means for transmitting an image through the distal section so that the image can be viewed at the viewing location, said image-transmitting means having an optical axis;
said image-transmitting means including an elongated image-transmitting rod for transmitting the image through at least a region of the distal section; and
said image-transmitting rod having a longitudinal axis and a wedge surface at a distal end of the rod which is at an acute angle with respect to the optical axis of the image-transmitting rod to provide an offset field of view.

2. An apparatus comprising:
a tubular body having a distal section and a proximal viewing location;
image-transmitting means in the tubular body having an optical axis for transmitting an image through the distal section so that the image can be viewed at the viewing location;
said image-transmitting means including a refracting prism forming the distal end of the image-transmitting means, said prism having a wedge surface at said distal end which is inclined at an acute angle relative to the optical axis to provide an offset field of view; and
said prism refracting the centerline of the field of view.

3. An apparatus comprising:
a body including an elongated tubular distal section adapted for insertion into a patient and a proximal viewing location;
image-transmitting means having an optical axis for transmitting an image through the distal section to the viewing location;
said image-transmitting means having an offset field of view and providing asymmetric distortion of the image; and
said image-transmitting means including means for at least reducing said distortion.

4. An apparatus as defined in claim 1 wherein said image-transmitting rod includes an objective of gradient index material and a relay rod of gradient index material, said relay rod being proximal of the objective and having a pitch length which is longer than the pitch length of the objective, said objective and said relay rod being at least partially within said distal section.

5. An apparatus as defined in claims 1, 2 or 4 including optical illumination fibers extending along at least a region of the image-transmitting means in the distal section of the body for conducting light to a region adjacent said wedge surface, said fibers having distal ends which lie at an acute angle with respect to said optical axis to direct the illumination generally toward the offset field of view.

6. An apparatus as defined in claims 1, 2, 4 or 5 wherein said acute angle of the wedge surface introduces distortion to the image and said image-transmitting means includes means for at least reducing said distortion.

7. An apparatus as defined in claims 3 or 6 wherein said body includes an eyepiece housing releasably coupled to said distal section, said distortion reducing means is in said eyepiece housing and said distal section is disposable.

8. An apparatus as defined in claims 3, 6 or 7 wherein said distortion includes compression of one side of the image and said distortion-reducing means includes means for differentially magnifying the image.

9. An apparatus as defined in claim 8 wherein said differential magnifying means includes at least one prism located proximally of said image-transmitting rod for differentially magnifying the image in one plane.

10. An apparatus as defined in claims 8 or 9 wherein said differential magnifying means includes first and second prisms having different indices of refraction and an interface for differentially magnifying the image in one plane.

11. An apparatus as defined in claim 10 wherein the first and second prisms have Abbe numbers within about 2 percent of each other, the first and second prisms elongate the field of view and said image-transmitting means includes means for shortening the elongated field of view.

12. An apparatus as defined in claims 1 or 5 wherein said image-transmitting rod includes an objective lens of gradient index material and a relay rod proximal of the objective lens and wherein the image-transmitting means includes means for enlarging the image from the relay rod.

*FIG. 1*

*FIG. 10*

*FIG. 3*

*FIG. 6*

*FIG. 2*

0 260 856

*FIG.4*

37b · 35 · 31 · 36 · 15b · 33 · 23b · 13b · 41 · 43

*FIG.5*

29c · X · 51 · 55c · 53c · 51 · 27c · 53 · 53 · 63c · 19c · 65c · *FIG.7*

w · N · N' · I · I' · 27 · u · 45 · 25

*FIG.8*

91 · 95 · 93 · 51 · 83 · 29d · 29d · X · 29d · 97 · 57 · 51 · 27d · 27d · 57 · 98 · 89 · 85 · 81 · 19d · 29d · 51 · 87 · 57

FIG. 9

FIG. 11

FIG. 12

FIG. 10

0 260 856